# EUROPEAN PATENT APPLICATION

(11) **EP 1 870 129 A1**
(43) Date of publication of application: **26.12.2007**
(21) Application number: 07252558.7
(22) Date of filing: 22.06.2007
(51) Int. Cl.: A61N 1/36, A61N 1/365

(54) **Sympathetic afferent activation for adjusting autonomic tone**

(30) Priority: 23.06.2006 US 473681
(71) Applicant: PACESETTER, INC., Sylmar, CA 91342-9221 (US)
(72) Inventor: Park, Euljoon, Valencia, CA 91381 (US); Farazi, Taranesh Ghaffari, San Jose, CA 95120 (US)
(74) Representative: Rees, David Christopher

(57) **Abstract**

An exemplary method includes delivering electrical stimulation to a sympathetic afferent nerve, acquiring information indicative of autonomic tone and, based at least in part on the information, determining if the delivering caused an increase in parasympathetic nerve activity. Various other exemplary methods, devices, systems, etc., are also disclosed.

## Description

Exemplary mechanisms presented herein generally relate to autonomic tone and activation of sympathetic afferent nerves. Various exemplary mechanisms are useful with cardiac pacing therapy.

The autonomic nervous system and the cardiovascular system are highly integrated whereby a change to one system generally affects the other system. The autonomic nervous system extends across the body and affects the cardiovascular system through both intracardiac and extracardiac mechanisms. Vasovagal (vasodepressor) syncope, which can be precipitated by unpleasant physical or emotional stimuli (e.g., pain, fright, sight of blood), is an example of autonomic and cardiovascular integration. Vasovagal syncope occurs fairly quickly and may be viewed as a short-term mechanism. Other natural mechanisms also act to counter excessive sympathetic activity and balance autonomic tone.

Various cardiac conditions are intimately associated with the autonomic nervous system and can impair or severely challenge such natural mechanisms. For example, congestive heart failure (CHF or simply HF) is characterized by increased sympathetic outflow and decreased parasympathetic outflow. HF has been associated with an elevated sympathetic tone and depressed parasympathetic tone (e.g., decreased activity from arterial and cardiopulmonary baroreceptors). However, blunted parasympathetic and arterial baroreflexes are not the sole mechanism for the high level of sympathetic activity in HF. It is well known that the cardiac sympathetic afferent reflex also contributes to an increase in sympathetic outflow. In this way, excitatory sympathetic reflexes initiated by hemodynamic changes and by the relative ischemia of HF may contribute to the observed increase in sympathetic efferent activity.

Such sympathetic effects are due to cardiac condition and viewed generally as long-term mechanisms. As described herein, implantable stimulation devices allow for exploitation of short-term sympathetic mechanism. In particular, various exemplary methods, devices, systems, etc., described herein aim to activate sympathetic afferent activity to thereby cause a desired response in parasympathetic activity. Other techniques are also disclosed.

According to the invention, there is provided a system comprising: means for delivering electrical stimulation to a sympathetic afferent nerve; means for acquiring information indicative of autonomic tone; and means for determining, based at least in part on the information, if the delivering caused an increase in parasympathetic nerve activity.

Preferably, the means for delivering delivers electrical stimulation to a left side sympathetic afferent nerve, or to a right side sympathetic afferent nerve. The electrical stimulation may be delivered to at least one nerve selected from superior cardiac nerves, middle cardiac nerves, inferior cardiac nerves, and thoracic cardiac nerves, and preferably to a nerve other than the left thoracic cardiac nerve. Preferably, the means for acquiring acquires heart rate and/or a measure of respiration and/or parasympathetic nerve activity.

The system preferably comprises an implantable cardiac electrical stimulation device, and the means for delivering comprises a pulse generator and the means for acquiring comprises sensing circuitry. Preferably, the device comprises: a processor, a memory and control logic, the control logic being implemented through use of the processor to call for the delivery of electrical stimulation to the sympathetic afferent nerve, to call for the acquiring of information indicative of autonomic tone and to determine, based at least in part on the information, if the delivering caused an increase in parasympathetic nerve activity. There may be connectors to electrically connect an electrode-bearing lead to the device. The lead may compromise a spiral electrode positionable on an autonomic nerve.

Preferably, the control logic is organised to adjust a cardiac pacing therapy based at least in part on whether the delivering caused an increase in parasympathetic nerve activity. It may be arranged to set a time period and if the determining means does not determine that the delivering caused an increase in parasympathetic nerve activity within the time period, then the control logic is arranged to adjust one or more stimulation parameters. Preferably. the stimulation parameter is selected from amplitude, frequency, duty cycle, stimulation site, and polarity.

The invention enables a method comprising: delivering electrical stimulation to a sympathetic afferent nerve; acquiring information indicative of autonomic tone; and based at least in part on the information, determining if the delivering caused an increase in parasympathetic nerve activity.

An exemplary method includes delivering electrical stimulation to a sympathetic afferent nerve, acquiring information indicative of autonomic tone and, based at least in part on the information, determining if the delivering caused an increase in parasympathetic nerve activity. Various other exemplary methods, devices, systems, etc., are also disclosed.

The invention may be carried into practice in varying ways and some embodiments will now be described with reference to accompanying drawings, in which:

Fig. 1 is a simplified diagram illustrating an exemplary implantable stimulation device in electrical communication with at least three leads implanted into a patient's heart and at least one other lead for sensing or delivering stimulation or shock therapy;

Fig. 2 is a functional block diagram of an exemplary implantable stimulation device illustrating basic elements that are configured to provide cardioversion, defibrillation, pacing stimulation or autonomic nerve stimulation or other tissue or nerve stimulation (the implantable stimulation device is further configured to sense information and administer stimulation pulses responsive to such information);

Fig. 3 is an approximate anatomical diagram illustrating an exemplary implantable stimulation device in electrical communication with an autonomic pathway and a block diagram of various cardiac plexuses associated with autonomic pathways;

Fig. 4 is a block diagram of an exemplary device and an exemplary method that can be implemented using the device;

Fig. 5 is a block diagram of an exemplary method for activating a sympathetic afferent nerve and determining whether the activating caused a parasympathetic response;

Fig. 6 is a block diagram of an exemplary method for classification how one or more sympathetic afferent nerves respond to stimulation;

Fig. 7 is a block diagram of an exemplary device and method for acquiring information indicative of autonomic tone;

Fig. 8 is an approximate anatomical diagram illustrating an exemplary implantable stimulation device in electrical communication with an autonomic pathway and a block diagram of exemplary method for acquiring information indicative of autonomic tone or parasympathetic nerve activity; and

Fig. 9 is a block diagram of an exemplary method for tracking changes in autonomic response following stimulation of sympathetic afferent nerve(s).

### Overview

An exemplary sensing and stimulation device is described followed by a description of the cardiovascular system. The description of autonomic nerve physiology includes relationships to cardiac plexuses and subplexuses. Various exemplary methods are described along with exemplary electrode configurations, devices and control logic.

### Exemplary Device

The techniques described below are intended to be implemented in connection with any stimulation device that is configured or configurable to sense and stimulate nerves and/or tissue, including stimulation of a patient's heart. While various examples refer to an implantable device, other examples are optionally implemented using an external device or a combination of internal and external components. For example, with respect to external devices, autonomic nerve activation has been achieved using external devices that deliver electromagnetic or magnetic radiation to a body (e.g., neck region, etc.). In another example, an external device for autonomic nerve activation may communicate with an implanted device (e.g., an implanted cardiac therapy device, etc.).

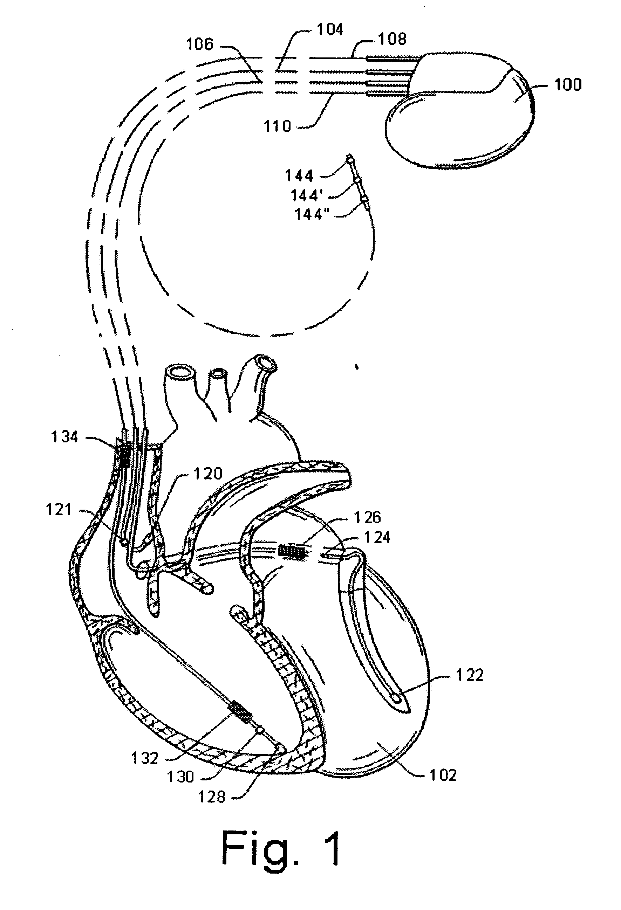

Fig. 1 shows an exemplary stimulation device 100 in electrical communication with a patient's heart 102 by way of three leads 104, 106, 108, suitable for delivering multi-chamber stimulation and shock therapy. The leads 104, 106, 108 are optionally configurable for delivery of stimulation pulses suitable for stimulation of autonomic nerves and/or for sensing autonomic nerve activity. In such configurations, the number of electrodes may vary from the number shown; electrode type may vary as well.

The device 100 includes a fourth lead 110 having, in this implementation, three electrodes 144, 144', 144" suitable for stimulation of tissue such as autonomic nerves and/or sensing physiologic signals (e.g., autonomic nerve activity) that may be used by the implanted system to modify therapy parameters. Such a lead is optional as a suitable device may have more or few leads than the device 100 shown in Fig. 1. The lead 110 may be positioned in and/or near a patient's heart or within a patient's body and remote from the heart.

The right atrial lead 104, as the name implies, is positioned in and/or passes through a patient's right atrium. The right atrial lead 104 may be used for sensing atrial cardiac signals, for providing right atrial chamber stimulation therapy and optionally for sensing autonomic nerve activity. As shown in Fig. 1, the stimulation device 100 is coupled to an implantable right atrial lead 104 having, for example, an atrial tip electrode 120, which typically is implanted in the patient's right atrial appendage. The lead 104, as shown in Fig. 1, also includes an atrial ring electrode 121. Of course, the lead 104 may have other electrodes as well. For example, the right atrial lead optionally includes a distal bifurcation having electrodes suitable for stimulation of autonomic nerves or other tissue or for sensing activity of autonomic nerves or other tissue.

To sense atrial cardiac signals, ventricular cardiac signals and/or to provide chamber pacing therapy, particularly on the left side of a patient's heart, the stimulation device 100 is coupled to a coronary sinus lead 106 designed for placement in the coronary sinus and/or tributary veins of the coronary sinus. Thus, the coronary sinus lead 106 may be used to position at least one distal electrode adjacent to the left ventricle and/or additional electrode(s) adjacent to the left atrium. In a normal heart, tributary veins of the coronary sinus include, but may not be limited to, the great cardiac vein, the left marginal vein, the left posterior ventricular vein, the middle cardiac vein, and the small cardiac vein.

Accordingly, an exemplary coronary sinus lead 106 may be used to receive atrial and ventricular cardiac signals and to deliver left ventricular pacing therapy using, for example, at least a left ventricular tip electrode 122, left atrial pacing therapy using at least a left atrial ring electrode 124, and shocking therapy using at least a left atrial coil electrode 126. For a complete description of an example of a coronary sinus lead, the reader is directed to U.S. Patent No. 5,466,254, "Coronary Sinus Lead with Atrial Sensing Capability" (Helland).

The coronary sinus lead 106 may further include electrodes for stimulation of autonomic nerves or for sensing autonomic nerve activity. For example, an exemplary coronary sinus lead includes pacing electrodes capable of delivering pacing pulses to a patient's left ventricle and at least one electrode capable of stimulating an autonomic nerve and/or sensing activity of an autonomic nerve. An exemplary coronary sinus lead (or left ventricular lead or left atrial lead) may also include at least one electrode on a bifurcation or leg of the lead.

Stimulation device 100 is also shown in electrical communication with the patient's heart 102 by way of an implantable right ventricular lead 108 having, in this exemplary implementation, a right ventricular tip electrode 128, a right ventricular ring electrode 130, a right ventricular (RV) coil electrode 132, and an SVC coil electrode 134. Typically, the right ventricular lead 108 is transvenously inserted into the heart 102 to place the right ventricular tip electrode 128 in the right ventricular apex so that the RV coil electrode 132 will be positioned in the right ventricle and the SVC coil electrode 134 will be positioned in the superior vena cava. Accordingly, the right ventricular lead 108 is capable of sensing or receiving cardiac signals, and delivering stimulation in the form of pacing and shock therapy to the right ventricle. An exemplary right ventricular lead may also include at least one electrode capable of stimulating an autonomic nerve and/or sensing activity of an autonomic nerve. Such an electrode may be positioned on the lead or a bifurcation or leg of the lead.

As already mentioned, more than one device may be used for performing various exemplary method described herein. For example, one device may operate to sense autonomic nerve activity while another device operates to delivery myocardial stimulation. In such an example, communication may occur from one device to the other or bi-directionally between the two devices. Communication may occur via telemetric circuit or by a circuit that emits energy into body tissue, at least some of the emitted energy receivable or detectable by the other device.

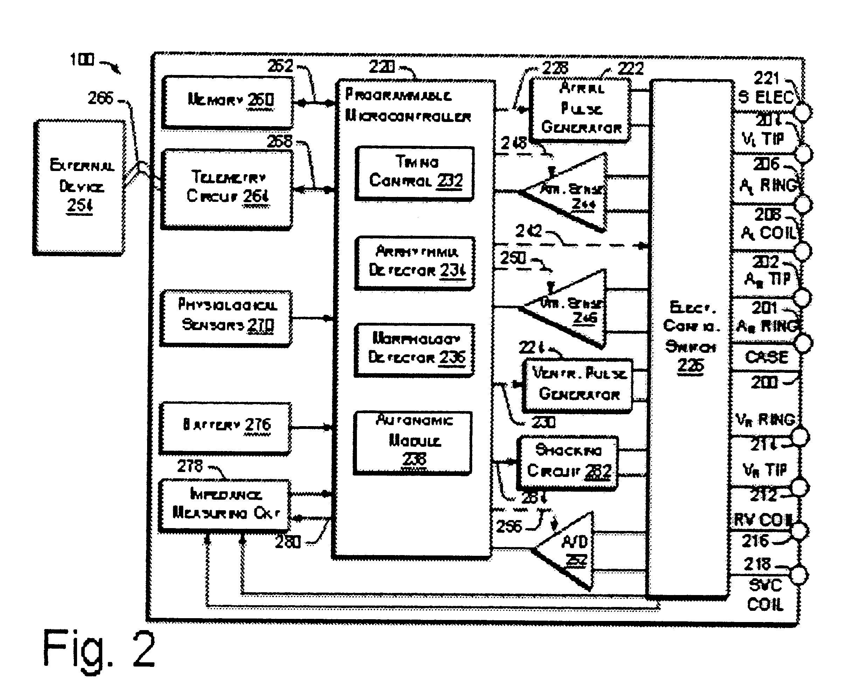

Fig. 2 shows an exemplary, simplified block diagram depicting various components of the device 100. The device 100 can be capable of treating both fast and slow arrhythmias with stimulation therapy, including cardioversion, defibrillation, and pacing stimulation. As described in more detail below, delivery of atrial anti-arrhythmia therapy may occur in response to classification of autonomic nerve activity.

While a particular multi-chamber device is shown, it is to be appreciated and understood that this is done for illustration purposes only. Thus, the techniques and methods described below can be implemented in connection with any suitably configured or configurable stimulation device. Accordingly, one of skill in the art could readily duplicate, eliminate, or disable the appropriate circuitry in any desired combination to provide a device capable of treating the appropriate chamber(s) or regions of a patient's heart with cardioversion, defibrillation, pacing stimulation and/or autonomic nerve stimulation.

Housing 200 for stimulation device 100 is often referred to as the "can", "case" or "case electrode", and may be programmably selected to act as the return electrode for all "unipolar" modes. Housing 200 may further be used as a return electrode alone or in combination with one or more of the coil electrodes 126, 132 and 134 for shocking purposes. Housing 200 further includes a connector (not shown) having a plurality of terminals 201, 202, 204, 206, 208, 212, 214, 216, 218, 221 (shown schematically and, for convenience, the names of the electrodes to which they are connected are shown next to the terminals).

To achieve right atrial sensing, pacing, autonomic nerve stimulation and/or autonomic nerve sensing, the connector includes at least a right atrial tip terminal (A_{R} TIP) 202 adapted for connection to the atrial tip electrode 120. A right atrial ring terminal (A_{R} RING) 201 is also shown, which is adapted for connection to the atrial ring electrode 121.

To achieve left chamber sensing, pacing, shocking, autonomic nerve stimulation and/or autonomic nerve sensing, the connector includes at least a left ventricular tip terminal (V_{L} TIP) 204, a left atrial ring terminal (A_{L} RING) 206, and a left atrial shocking terminal (A_{L} COIL) 208, which are adapted for connection to the left ventricular tip electrode 122, the left atrial ring electrode 124, and the left atrial coil electrode 126, respectively. Connection to other suitable tissue stimulation electrodes is also possible via these and/or other terminals (e.g., via a stimulation/sensing terminal S ELEC 221). In general, the stimulation/sensing terminal S ELEC 221 may be used for any of a variety of tissue activation or tissue sensing. An exemplary device may include one or more such terminals for purposes of stimulation and/or sensing.

To support right chamber sensing, pacing, shocking, autonomic nerve stimulation and/or autonomic nerve sensing, the connector further includes a right ventricular tip terminal (V_{R} TIP) 212, a right ventricular ring terminal (V_{R} RING) 214, a right ventricular shocking terminal (RV COIL) 216, and a superior vena cava shocking terminal (SVC COIL) 218, which are adapted for connection to the right ventricular tip electrode 128, right ventricular ring electrode 130, the RV coil electrode 132, and the SVC coil electrode 134, respectively.

At the core of the stimulation device 100 is a programmable microcontroller 220 that controls the various modes of stimulation therapy. As is well known in the art, microcontroller 220 typically includes a microprocessor, or equivalent control circuitry, designed specifically for controlling the delivery of various therapies, and may further include RAM or ROM memory, logic and timing circuitry, state machine circuitry, and I/O circuitry. Typically, microcontroller 220 includes the ability to process or monitor input signals (data or information) as controlled by a program code stored in a designated block of memory. The type of microcontroller is not critical to the described implementations. Rather, any suitable microcontroller 220 may be used that carries out the functions described herein. The use of microprocessor-based control circuits for performing timing and data analysis functions are well known in the art.

Representative types of control circuitry that may be used in connection with the described embodiments can include the microprocessor-based control system of U.S. Patent No. 4,940,052 (Mann et al.), the state-machine of U.S. Patent Nos. 4,712,555 (Thornander) and 4,944,298 (Sholder). For a more detailed description of the various timing intervals used within the stimulation device and their inter-relationship, see U.S. Patent 4,788,980 (Mann et al.).

Fig. 2 also shows an atrial pulse generator 222 and a ventricular pulse generator 224 that generate pacing stimulation pulses for delivery by the right atrial lead 104, the coronary sinus lead 106, and/or the right ventricular lead 108 via an electrode configuration switch 226. It is understood that in order to provide stimulation therapy in each of the four chambers of the heart (or for other tissue activation) the atrial and ventricular pulse generators, 222 and 224, may include dedicated, independent pulse generators, multiplexed pulse generators, or shared pulse generators. The pulse generators 222 and 224 are controlled by the microcontroller 220 via appropriate control signals 228 and 230, respectively, to trigger or inhibit the stimulation pulses.

Microcontroller 220 further includes timing control circuitry 232 to control the timing of the stimulation pulses (e.g., pacing rate, atrio-ventricular (AV) delay, interatrial conduction (AA) delay, or interventricular conduction (VV) delay, etc.) as well as to keep track of the timing of refractory periods, blanking intervals, noise detection windows, evoked response windows, alert intervals, marker channel timing, etc., which is well known in the art.

Microcontroller 220 further includes an arrhythmia detector 234 and optionally an orthostatic compensator and/or a minute ventilation (MV) response module, the latter are not shown in Fig. 2. These components can be utilized by the stimulation device 100 for determining desirable times to administer various therapies, including those to reduce the effects of orthostatic hypotension. The aforementioned components may be implemented in hardware as part of the microcontroller 220, or as software/firmware instructions programmed into the device and executed on the microcontroller 220 during certain modes of operation.

Microcontroller 220 further includes a morphology discrimination module 236. This module is optionally used to implement various exemplary recognition algorithms. For example, the module 236 may include algorithms for recognition of certain characteristics in autonomic nerve activity, as described in more detail below. The aforementioned components may be implemented in hardware as part of the microcontroller 220, or as software/firmware instructions programmed into the device and executed on the microcontroller 220 during certain modes of operation.

Microcontroller 220 further includes an autonomic module 238 for performing a variety of tasks related to autonomic nerve sensing and/or stimulation. This component may also be utilized by the device 100 for determining desirable times to administer various therapies (e.g., atrial anti-arrhythmia therapies). The module 238 may be implemented in hardware as part of the microcontroller 220 or as software/firmware instructions programmed into the device and executed on the microcontroller 220 during certain modes of operation.

The electronic configuration switch 226 includes a plurality of switches for connecting the desired electrodes to the appropriate I/O circuits, thereby providing complete electrode programmability. Accordingly, switch 226, in response to a control signal 242 from the microcontroller 220, determines the polarity of the stimulation pulses (e.g., unipolar, bipolar, etc.) by selectively closing the appropriate combination of switches (not shown) as is known in the art. Similarly, the switch 226 may configure or select electrodes for sensing.

Atrial sensing circuits 244 and ventricular sensing circuits 246 may also be selectively coupled to the right atrial lead 104, coronary sinus lead 106, the right ventricular lead 108, and the lead 110 through the switch 226 for any of a variety of purposes (e.g., detecting the presence of cardiac activity in each of the four chambers of the heart, sensing autonomic nerve activity, etc.). Accordingly, the atrial (ATR. SENSE) and ventricular (VTR. SENSE) sensing circuits, 244 and 246, may include dedicated sense amplifiers, multiplexed amplifiers, or shared amplifiers. Switch 226 can determine the "sensing polarity" of the cardiac signal by selectively closing the appropriate switches, as is also known in the art. In this way, the clinician may program the sensing polarity independent of the stimulation polarity. The sensing circuits (e.g., 244 and 246) are optionally capable of obtaining information indicative of tissue capture.

Each sensing circuit 244 and 246 preferably employs one or more low power, precision amplifiers with programmable gain and/or automatic gain control, bandpass filtering, and a threshold detection circuit, as known in the art, to selectively sense the cardiac or autonomic nerve signal of interest. The automatic gain control enables the device 100 to deal effectively with the difficult problem of sensing the low amplitude signal characteristics of atrial or ventricular fibrillation. Such gain control may aid in sensing of other signals (e.g., autonomic nerve, etc.).

The outputs of the atrial and ventricular sensing circuits 244 and 246 are connected to the microcontroller 220, which, in turn, is able to trigger or inhibit the atrial and ventricular pulse generators 222 and 224, respectively, in a demand fashion in response to the absence or presence of cardiac activity in the appropriate chambers of the heart. Furthermore, as described herein, the microcontroller 220 is also capable of analyzing information output from the sensing circuits 244 and 246 and/or the data acquisition system 252 to determine or detect whether and to what degree tissue capture has occurred and to program a pulse, or pulses, in response to such determinations. The sensing circuits 244 and 246, in turn, receive control signals over signal lines 248 and 250 from the microcontroller 220 for purposes of controlling the gain, threshold, polarization charge removal circuitry (not shown), and the timing of any blocking circuitry (not shown) coupled to the inputs of the sensing circuits, 244 and 246, as is known in the art.

For arrhythmia detection, the device 100 may utilize the atrial and ventricular sensing circuits, 244 and 246, to sense cardiac signals to determine whether a rhythm is physiologic or pathologic. Of course, other sensing circuits may be available depending on need and/or desire. In reference to arrhythmias, as used herein, "sensing" is reserved for the noting of an electrical signal or obtaining data (information), and "detection" is the processing (analysis) of these sensed signals and noting the presence of an arrhythmia or of a precursor or other factor that may indicate a risk of or likelihood of an imminent onset of an arrhythmia. Various exemplary techniques described herein pertain to classification of autonomic nerve activity with respect to atrial behavior. Such techniques rely on sensed information and can detect or aid in detection of an arrhythmia or of a precursor or other factor that may indicate a risk of or likelihood of an imminent onset of an arrhythmia. Thus, the module 234 may rely, where appropriate, on the autonomic module 238.

Such an exemplary detection module 234, optionally uses timing intervals between sensed events (e.g., P-waves, R-waves, and depolarization signals associated with fibrillation which are sometimes referred to as "F-waves" or "Fib-waves") and to perform one or more comparisons to a predefined rate zone limit (i.e., bradycardia, normal, low rate VT, high rate VT, and fibrillation rate zones) and/or various other characteristics (e.g., sudden onset, stability, physiologic sensors, and morphology, etc.) in order to determine the type of remedial therapy (e.g., anti-arrhythmia, etc.) that is desired or needed (e.g., bradycardia pacing, anti-tachycardia pacing, cardioversion shocks or defibrillation shocks, collectively referred to as "tiered therapy"). Similar rules can be applied to the atrial channel to determine if there is an atrial tachyarrhythmia or atrial fibrillation with appropriate classification and intervention. Such a module is optionally suitable for performing various exemplary methods described herein. For example, such a module optionally allows for analyses related to action potentials (e.g., MAPs, T waves, etc.) and characteristics thereof (e.g., alternans, activation times, repolarization times, derivatives, etc.).

Cardiac signals and/or other signals are typically applied to inputs of an analog-to-digital (A/D) data acquisition system 252. For example, the data acquisition system 252 can be configured to acquire intracardiac electrogram signals, convert the raw analog data into a digital signal, and store the digital signals for later processing and/or telemetric transmission to an external device 254. The data acquisition system 252 is coupled to the right atrial lead 104, the coronary sinus lead 106, the right ventricular lead 108 and/or the lead 110 lead through the switch 226 to sample cardiac signals or other signals across any pair of desired electrodes.

The microcontroller 220 is further coupled to a memory 260 by a suitable data/address bus 262, wherein the programmable operating parameters used by the microcontroller 220 are stored and modified, as required, in order to customize the operation of the stimulation device 100 to suit the needs of a particular patient. Such operating parameters define, for example, pacing pulse amplitude, pulse duration, electrode polarity, rate, sensitivity, automatic features, arrhythmia detection criteria, and the amplitude, waveshape, number of pulses, and vector of each shocking pulse to be delivered to the patient's heart 102 within each respective tier of therapy. One feature of the described embodiments is the ability to sense and store a relatively large amount of data (e.g., from the data acquisition system 252), which data may then be used for subsequent analysis to guide the programming of the device.

Advantageously, the operating parameters of the implantable device 100 may be non-invasively programmed into the memory 260 through a telemetry circuit 264 in telemetric communication via communication link 266 with the external device 254, such as a programmer, transtelephonic transceiver, or a diagnostic system analyzer. The microcontroller 220 activates the telemetry circuit 264 with a control signal 268. The telemetry circuit 264 advantageously allows intracardiac electrograms, status information and/or other information relating to the operation of the device 100 (as contained in the microcontroller 220 or memory 260) to be sent to the external device 254 through an established communication link 266.

The stimulation device 100 can further includes one or more physiologic sensors 270. For example, a physiologic sensor commonly referred to as a "rate-responsive" sensor is optionally included and used to adjust pacing stimulation rate according to the exercise state of the patient. However, one or more of the physiologic sensors 270 may further be used to detect changes in cardiac output (see, e.g., U.S. Pat. No. 6,314,323, entitled "Heart stimulator determining cardiac output, by measuring the systolic pressure, for controlling the stimulation", to Ekwall, issued November 6, 2001, which discusses a pressure sensor adapted to sense pressure in a right ventricle and to generate an electrical pressure signal corresponding to the sensed pressure, an integrator supplied with the pressure signal which integrates the pressure signal between a start time and a stop time to produce an integration result that corresponds to cardiac output), changes in the physiological condition of the heart, diurnal changes in activity (e.g., detecting sleep and wake states), etc. Accordingly, the microcontroller 220 responds by adjusting the various pacing parameters (such as rate, AV Delay, VV Delay, etc.) at which the atrial and ventricular pulse generators, 222 and 224, generate stimulation pulses.

While shown as being included within the stimulation device 100, it is to be understood that the physiologic sensor 270 may also be external to the stimulation device 100, yet still be implanted within or carried by the patient. Examples of physiologic sensors that may be implemented in device 100 include known sensors that, for example, sense pressure, respiration rate, pH of blood, ventricular gradient, cardiac output, preload, afterload, contractility, and so forth. Another sensor that may be used is one that detects activity variance, wherein an activity sensor is monitored diurnally to detect the low variance in the measurement corresponding to the sleep state. For a complete description of the activity variance sensor, the reader is directed to U.S. Patent No. 5,476,483 (Bornzin et al.), issued 12/19/1995, which patent is hereby incorporated by reference.

Various exemplary methods, devices, systems, etc., described herein optionally use such a pressure transducer to measure pressures in the body (e.g., chamber of heart, vessel, etc.). The company, Radi Medical Systems AB (Uppsala, Sweden), markets various lead-based sensors for intracoronary pressure measurements, coronary flow reserve measurements and intravascular temperature measurements. Such sensor technologies may be suitably adapted for use with an implantable device for in vivo measurements of physiology.

The companies Nellcor (Pleasanton, California) and Masimo Corporation (Irvine, California) market pulse oximeters that may be used externally (e.g., finger, toe, etc.). Where desired, information from such external sensors may be communicated wirelessly to the implantable device, for example, via an implantable device programmer. Other sensors may be implantable and suitably connected to or in communication with the exemplary implantable device 100. Technology exists for lead-based oximeters. For example, a study by Tsukada et al., "Development of catheter-type optical oxygen sensor and applications to bioinstrumentation," Biosens Bioelectron, 2003 Oct 15; 18(12): 1439-45, reported use of a catheter-type optical oxygen sensor based on phosphorescence lifetime.

Various photoplethysmography techniques suitable for use with an implantable device such as the device 100 are disclosed in U.S. Patent No. 6,491,639 (Turcott), issued 12/10/2002 and U.S. Patent No. 6,731,967 (Turcott), issued 5/4/2004. The exemplary implantable device 100 may include or operate in conjunction with one or more PPG sensors in a can-based, lead-based or other manner whereby PPG information is communicated to the device. Such sensors may determine SaO₂, SvO₂ or other oxygen-related parameters. Other sensors suitable for use with the exemplary device 100 include cardiomechanical sensors (CMEs).

The one or more physiologic sensors 270 optionally include a position and/or movement sensor mounted within the housing 200 of the stimulation device 100 to detect movement in the patient's position or the patient's position. Such a sensor may operate in conjunction with a position and/or movement analysis module (e.g., executable in conjunction with the microcontroller 220). The position and/or movement sensor may be implemented in many ways. In one particular implementation, the position sensor is implemented as an accelerometer-based sensor capable of measuring acceleration, position, etc. For example, such a sensor may be capable of measuring dynamic acceleration and/or static acceleration. In general, movement of the patient will result in a signal from the accelerometer. For example, such an accelerometer-based sensor can provide a signal to the microcontroller 220 that can be processed to indicate that the patient is undergoing heightened physical exertion, moving directionally upwards or downwards, etc.

Further, depending on position of the implanted device and such a movement sensor, the sensor may measure or monitor chest movement indicative of respiratory characteristics. For example, for a typical implant in the upper chest, upon inspiration, the upper chest expands thereby causing the implanted device to move. Accordingly, upon expiration, the contraction of the upper chest causes the device to move again. Such a movement sensor may sense information capable of distinguishing whether a patient is horizontal, vertical, etc.

While respiratory information may be obtained via the one or more physiologic sensors 270, a minute ventilation (MV) sensor may sense respiratory information related to minute ventilation, which is defined as the total volume of air that moves in and out of a patient's lungs in a minute. A typical MV sensor uses thoracic impedance, which is a measure of impedance across the chest cavity wherein lungs filled with air have higher impedance than empty lungs. Thus, upon inhalation, impedance increases; whereas upon exhalation, impedance decreases. Of course, a thoracic impedance (e.g., intrathoracic impedance) may be used to determine tidal volume or measures other than minute ventilation.

With respect to impedance measurement electrode configurations, a right ventricular tip electrode and case electrode may provide current while a right ventricular ring electrode and case electrode may allow for potential sensing. Of course, other configurations and/or arrangements may be used to acquire measurements over other paths (e.g., a superior-inferior path and a left-right path, etc.). Multiple measurements may be used wherein each measurement has a corresponding path.

Direct measurement of autonomic nerve activity (e.g., vagal nerve or sympathetic nerve) may be achieved using a cuff or other suitable electrode appropriately positioned in relationship to an autonomic nerve. Nerve signals are typically of amplitude measured in microvolts (e.g., less than approximately 30 microvolts). Sensing may be coordinated with other events, whether natural event or events related to some form of stimulation therapy. As discussed herein, some degree of synchronization may occur between calling for and/or delivering stimulation for autonomic nerve activation and sensing of neural activity.

Signals generated by the one or more physiologic sensors 270 (e.g., MV sensor, impedance sensor, blood pressure, etc.) are optionally processed by the microcontroller 220 in determining whether to apply one or more therapies. More specifically, with respect to a movement sensor, the microcontroller 220 may receive a signal from an accelerometer-based sensor that may be processed to produce an acceleration component along a vertical axis (i.e., z-axis signal). This acceleration component may be used to determine whether there is an increased or decreased level of activity in the patient, etc. The microcontroller 220 optionally integrates such a signal over time to produce a velocity component along the vertical direction. The vertical velocity may be used to determine a patient's position/activity aspects as well, such as whether the patient is going upstairs or downstairs. If the patient is going upstairs, the microcontroller 220 may increase the pacing rate or invoke an orthostatic compensator to apply a prescribed stimulation therapy, especially at the onset. If the patient is traversing downstairs, the device might decrease a pacing rate or perhaps invoke a MV response module (e.g., operational with the microcontroller 220) to control one or more therapies during the descent. The MV response module may provide information to be used in determining a suitable pacing rate by, for example, measuring the thoracic impedance from a MV sensor, computing the current MV, and comparing that with a long-term average of MV.

The microcontroller 220 can also monitor one or more of the sensor signals for any indication that the patient has moved from a supine position to a prone or upright position. For example, the integrated velocity signal computed from the vertical acceleration component of the sensor data may be used to determine that the patient has just stood up from a chair or sat up in bed. A sudden change in the vertical signal (e.g., a positive change in a direction normal to the surface of the earth), particularly following a prolonged period with little activity while the patient is sleeping or resting, confirms that a posture-changing event occurred. The microcontroller 220 optionally uses this information as one potential condition for deciding whether to invoke, for example, an orthostatic compensator to apply cardiac pacing therapy for treating orthostatic hypotension. Other possible uses also exist with respect to autonomic nerve activation for blood pressure control or for other purposes.

While a two-axis accelerometer may adequately detect tilt with respect to acceleration of gravity, the exemplary stimulation device 100 may also or alternatively be equipped with a GMR (giant magnetoresistance) sensor and circuitry that detects the earth's magnetic fields. Such a GMR sensor and circuitry may be used to ascertain absolute orientation coordinates based on the earth's magnetic fields. The device is thus able to discern a true vertical direction regardless of the patient's position (i.e., whether the patient is lying down or standing up). Where three-axes are measured by various sensors, coordinates may then be taken relative to the absolute orientation coordinates from the GMR. For instance, as a person sits up, the axial coordinates of an accelerometer-based sensor might change by 90°, but the sensor signals may be calibrated as to the true vertical direction based on the output of a GMR sensor and circuitry.

The stimulation device additionally includes a battery 276 that provides operating power to all of the circuits shown in Fig. 2. For the stimulation device 100, which can employ shocking therapy, the battery 276 is capable of operating at low current drains for long periods of time (e.g., preferably less than 10 µA), and is capable of providing high-current pulses (for capacitor charging) when the patient requires a shock or other stimulation pulse, for example, according to various exemplary methods, systems and/or devices described below. The battery 276 also desirably has a predictable discharge characteristic so that elective replacement time can be determined a priori or detected.

The stimulation device 100 can further include magnet detection circuitry (not shown in Fig. 2), coupled to the microcontroller 220, to detect when a magnet is placed over the stimulation device 100. A magnet may be used by a clinician to perform various test functions of the stimulation device 100 and/or to signal the microcontroller 220 that the external programmer 254 is in place to receive or transmit data to the microcontroller 220 through the telemetry circuits 264.

The stimulation device 100 further includes an impedance measuring circuit 278 that is enabled by the microcontroller 220 via a control signal 280. The impedance measuring circuit 278 may operate with an impedance sensor included as one of the physiological sensors 270. The known uses for an impedance measuring circuit 278 include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 278 is advantageously coupled to the switch 226 so that any desired electrode may be used.

The impedance measuring circuit 278 may also measure impedance related to lung inflation. Such a circuit may use a case electrode, an electrode positioned in or proximate to the heart and/or another electrode positioned within or proximate to the chest cavity. Various exemplary methods described below optionally rely on impedance measurements to determine lung inflation, inspiratory vagal excitation, which can inhibit excitatory signals to various muscles (e.g., diaphragm, external intercostals, etc.), or blood pressure (e.g., via relationship between vessel size due to blood pressure changes, etc.).

In the case where the stimulation device 100 is intended to operate as an implantable cardioverter/defibrillator (ICD) device, it detects the occurrence of an arrhythmia, and automatically applies an appropriate therapy to the heart aimed at terminating the detected arrhythmia and converting the heart back to a normal sinus rhythm. To this end, the microcontroller 220 further controls a shocking circuit 282 by way of a control signal 284. Shocking circuit 282 is presented as an example herein as other exemplary circuits are discussed below for charging and/or discharging stored charge.

In this example, the shocking circuit 282 can generate shocking or stimulation pulses of low (e.g., up to 0.5 J), moderate (e.g., 0.5 J to 10 J), or high energy (e.g., 11 J to 40 J), as controlled by the microcontroller 220. Such shocking pulses are applied to the patient's heart 102 through at least two electrodes, and as shown in this embodiment, selected from the left atrial coil electrode 126, the RV coil electrode 132, and/or the SVC coil electrode 134. As noted above, the housing 200 may act as an active electrode in combination with the RV electrode 132, or as part of a split electrical vector using the SVC coil electrode 134 or the left atrial coil electrode 126 (i.e., using the RV electrode as a common electrode).

Cardioversion level shocks are generally considered to be of low to moderate energy level (so as to minimize pain felt by the patient), and/or synchronized with an R-wave and/or pertaining to the treatment of tachycardia. Defibrillation shocks are generally of moderate to high energy level (e.g., corresponding to thresholds in the range of approximately 5 J to approximately 40 J), typically delivered synchronously , though R-waves may be disorganized, and pertaining exclusively to the treatment of fibrillation or fast polymorphic VT (e.g., ventricular fibrillation, which is discussed in more detail below).
Accordingly, the microcontroller 220 is capable of controlling the synchronous or asynchronous delivery of the shocking pulses.

As already mentioned, the device 100 of Figs. 1 and 2 has various features suitable for sensing autonomic nerve activity and calling for and delivering energy for myocardial and/or autonomic nerve activation. With respect to autonomic nerves, the module 238 may be used together with any of the various pulse generators, electrodes, etc. In general, autonomic nerve activation involves direct or indirect nerve stimulation and/or transvenous nerve stimulation. Such stimulation may aim to stimulate autonomic nerves distant from the heart or proximate to the heart, including cardiac plexuses or subplexuses. The term plexus includes subplexus. Some plexuses are referred to at times as "fat pads" (e.g., surrounded by fatty tissue).

The description of the device 100 of Figs. 1 and 2 makes various references to autonomic nerves; however, such a device may be used for other nerve sensing and/or stimulation (CNS, etc.). For example, some afferent autonomic nerves transmit information from the periphery to the CNS. In addition, some afferent autonomic nerves interact with the CNS concerned with the mediation of visceral sensation and the regulation of vasomotor and respiratory reflexes, for example the baroreceptors and chemoreceptors in the carotid sinus and aortic arch which are important in the control of heart rate, blood pressure and respiratory activity. These afferent fibres are usually carried to the CNS by major autonomic nerves such as the vagus, splanchnic or pelvic nerves, although afferent pain nerve fibers from blood vessels may be carried by somatic nerves. Various sympathetic afferent pathways are discussed below.

Various exemplary methods, devices, systems, etc., include mechanisms for classifying information carried by autonomic nerves. In particular, an exemplary method may include sensing nerve activity in one or more autonomic pathways and classifying such activity as efferent or afferent activity. An exemplary controller may then call for a particular action based at least in part on a classification of the autonomic nerve activity. For example, sympathetic afferent stimulation may result in immediate sympathetic efferent nerve activity and delayed parasympathetic afferent and/or efferent nerve activity. An implantable device may call for one or more actions in response to efferent and/or afferent nerve activity. Other examples are discussed below.

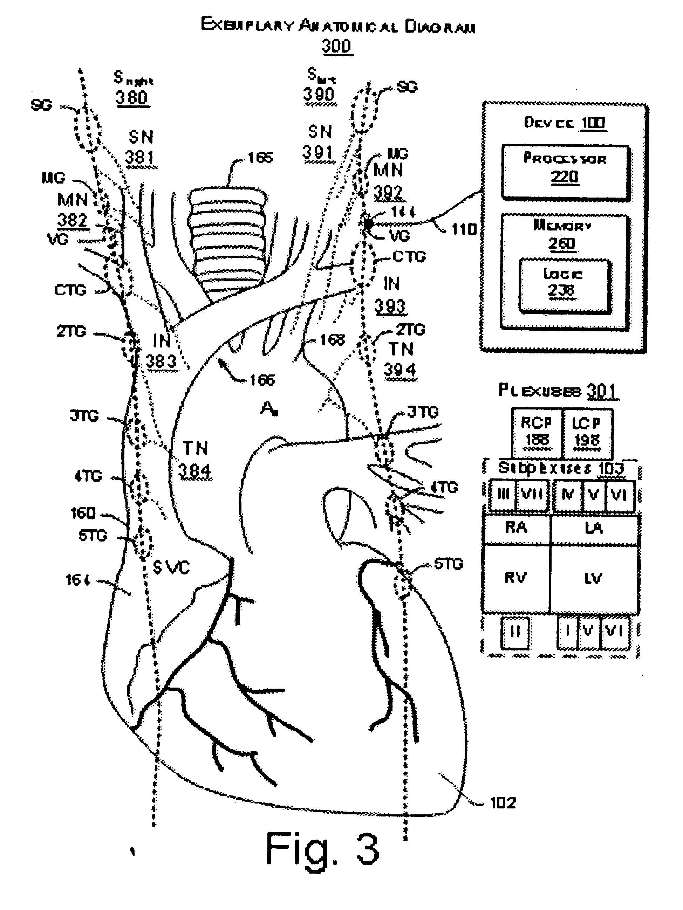

To understand better particular examples of sensing, classifying and controlling, Fig. 3 shows an approximate anatomical diagram 300 and a more generalized block diagram of plexuses associated with the heart 301. An exemplary device 100 includes a processor 220, memory 260 and logic 238 (see, e.g., device 100 of Fig. 2), which may be stored in the memory 260. The device 100 also includes a lead 110 and an electrode(s) positioned to stimulate an autonomic pathway.

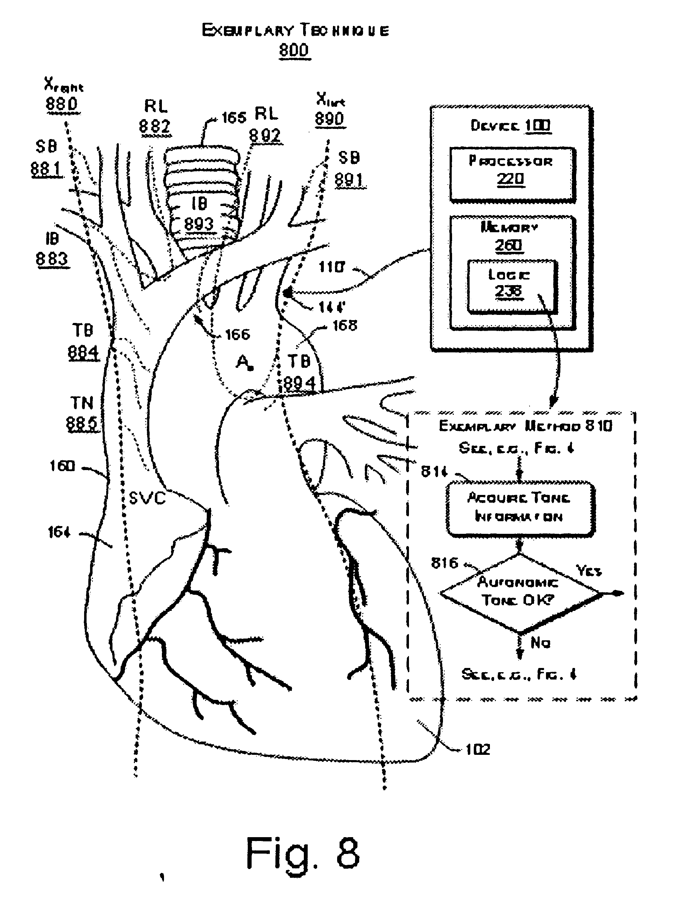

Fig. 3 shows various sympathetic pathways while Fig. 8, described further below, shows various parasympathetic pathways. The pathways presented in Figs. 3 and 8 may be used for selecting sites for sensing nerve activity and/or sites for nerve stimulating. Epicardial and/or endocardial sites for sensing and/or stimulating may be selected in part with reference to Fig. 1, 3, 8 or with reference to an article by Kawashima ("The autonomic nervous system of the human heart with special reference to its origin, course, and peripheral distribution", Anat Embryol. (2005) 209: 425-438) or an article by Pauza et al. ("Morphology, distribution, and variability of the epicardiac neural ganglionated subplexuses in the human heart", The Anatomical Record (2000) 259(4): 353-382). Of course, for an individual patient, imaging modalities (MR, CT, etc.) may identify sites or sites may be identified through use of one or more invasive techniques (e.g., surgical, catheter, etc.).

The diagram 300 of Fig. 3 includes the heart 102, other structures and various sympathetic pathways. In particular, the diagram 300 illustrates a right sympathetic branch 380 (S_{right}) and a left sympathetic branch 390 (S_{left}). Each of the right and left branches include ganglia such as a superior cervical ganglion (SG), a middle cervical ganglion (MG), a vertebral ganglion (VG), a cerviocothoracic (stellate) ganglion (CTG), and various thoracic ganglion (e.g., 2TG-5TG). Various cardiac nerves arise from the right sympathetic branch 380 and from the left sympathetic branch 390. These cardiac nerves include the left and right superior cardiac nerves 381, 391 (SN), the left and right middle cardiac nerves 382, 392 (MN), the right and left inferior cardiac nerves 383, 393 (IN) and the right and left thoracic cardiac nerves 384, 394 (TN). Noting that some subjects do not include all of the aforementioned ganglia or cardiac nerves. Further, the dashed lines do not indicate any particular length but rather a general course of such branches as they extend to, or around, the heart and other structures

The vagal nerve is part of the autonomic system and regarded primarily as a parasympathetic nerve and is described in more detail with respect to Fig. 8. Various autonomic nerve bundles and plexuses exist that include a mixture of parasympathetic and sympathetic nerves.

Referring to the block diagram of cardiac plexuses 301, according to Kawashima, the cardiac plexus includes the right cardiac plexus 188 (RCP), which usually surrounds the brachiocephalic trunk 166 (also known as the innominate artery), and the left cardiac plexus 198 (LCP), which surrounds the aortic arch 168. On the right side, Kawashima observed several autonomic nerves passing through the dorsal, rather than the ventral, aspect of the aortic arch while, on the left side, no differences between the ventral and dorsal courses to the aortic arch were observed.

Various nerves identified in the Kawashima article extend to one or more epicardial autonomic plexuses, also referred to herein as subplexuses 103. The aforementioned article by Pauza et al., reports that the epicardial plexus includes seven subplexuses: (I) left coronary, (II) right coronary, (III) ventral right atrial, (IV) ventral left atrial, (V) left dorsal, (VI) middle dorsal, and (VII) dorsal right atrial. The Pauza article states that, in general, the human right atrium is innervated by two subplexuses (III, VII), the left atrium by three subplexuses (IV, V, VI), the right ventricle by one subplexus (II), and the left ventricle by three subplexuses (I, V, VI). The Pauza article also notes that diagrams from Mizeres ("The cardiac plexus in man", Am. J. Anat. (1963) 112:141-151), suggest that "left epicardiac subplexuses may be considered as being formed by nerves derived from the left side of the deep extrinsic cardiac plexus, whereas ventral and dorsal right atrial subplexuses should be considered as being supplied by preganglionated nerves extending from the right vagus nerve and right sympathetic trunk, as their branches course in the adventitia of the right pulmonary artery and superior vena cava". The Pauza article also states that the left coronary (I), right coronary (II), ventral left atrial (IV) and middle dorsal (VI) subplexuses "may be considered as being formed by the deep extrinsic plexus that receives equally from both vagi and sympathetic trunks".

The RCP 188 and the LCP 198 are in communication with the subplexuses 103, where the subplexuses specifically identified with atrial activity are shown adjacent the right and left atria (e.g., right atrial subplexuses III and VII and left atrial subplexuses IV, V and VI) and the subplexuses specifically identified with ventricular activity are shown adjacent the right and left ventricles (e.g., the right ventricular subplexus II and the left ventricular subplexuses I, V and VI, noting some overlap with the left atrium). The subplexuses are referred to as "epicardial" subplexuses, which innervate the heart 102 to some extent.

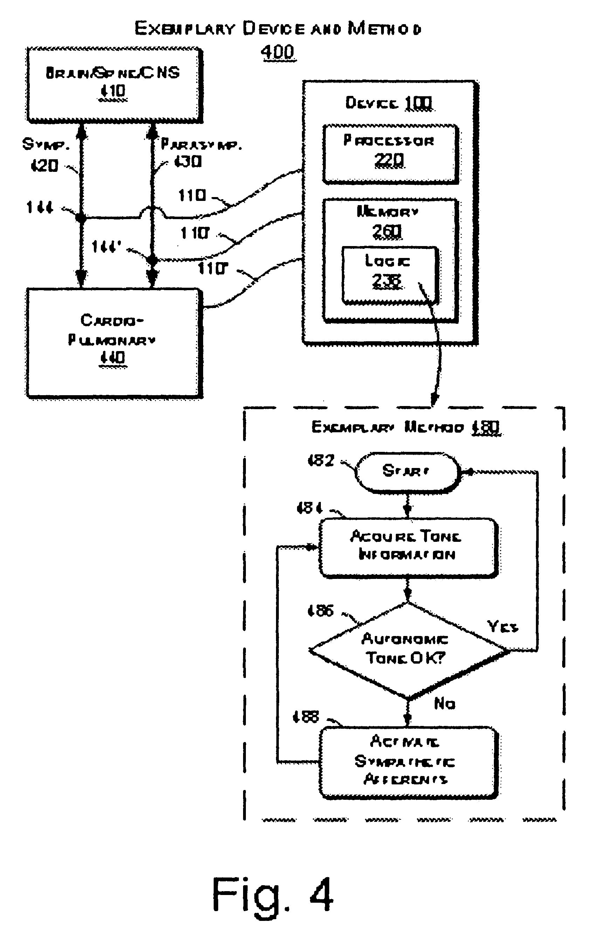

Fig. 4 shows an exemplary device and method 400 that can activate sympathetic afferent pathways. The device 100 and method 480 are shown in conjunction with an anatomical block diagram that includes the brain/spine/CNS 410, the cardiopulmonary system 440, sympathetic pathways 420 and parasympathetic pathways 430.

While some autonomic pathways may operate without direct communication to the brain, the brain often activates efferent pathways and receives information via afferent pathways. Feedback can be positive and/or negative. Consider an example where ischemia occurs. Depending upon the location and extent of ischemia there can either be vasodepressor reflex responses consisting of decreases in blood pressure, heart rate, bradyarrhythmias and nausea/vomiting or excitatory responses that include tachyarrhythmias, hypertension and angina pectoris. The former responses are mainly a function of parasympathetic afferents while the latter appear to be caused by activation of sympathetic afferents. In another example, consider that epicardial electrical stimulation of the central end of a left cardiac sympathetic nerve (rat model) blunted the baroreflex.

As an example of positive and negative feedback consider that distension of the aorta excites sympathetic afferent fibers that cause an increase in arterial blood pressure due to increased sympathetic outflow to the heart and blood vessels. The reflex center for this positive feedback mechanism is located in the spinal cord and, when the reflex is activated, it can modulate other negative feedback control systems. Provided an increase in sympathetic afferent activity, negative feedback may decrease sympathetic tone and/or increase parasympathetic tone. As described herein, stimulation of sympathetic afferent nerves can affect parasympathetic tone, directly or indirectly.

With respect to the brain, cardiopulmonary parasympathetic afferents have a central synapse in the nucleus tractus solitarii (NTS), which may receive convergent inputs from sympathetic and parasympathetic afferents that may interact in an occlusive manner. However, stimulation of cardiac sympathetic and parasympathetic branches (stimulated electrically at 1 Hz in a feline model), either separately or in combination, demonstrated that some parasympathetic and sympathetic afferent inputs could be additive or facilitative.

The NTS is not the only structure involved with autonomic nerve processing as increased activity of paraventricular nucleus (PVN) neurons, which occurs in heart failure, likely reflects the compromised state of cardiovascular afferent systems, whether at the sensory ending, in the afferent fiber, or in the hindbrain processing of the afferent signal.

With respect to other areas of the brain in relationship to specific nerve activity, structures associated with cardiac parasympathetic nerves (e.g., preganglionic neurons) include two locations in the medulla oblongata: the nucleus ambiguous (NA) and the dorsal vagal motor nucleus (DVMN). A study in the rat brain (Jones, "Vagal control of the rat heart", Experimental Physiology (2001) 86.6, 797-801) found that neurons of the ventral group near the NA have a discharge pattern which reflects strong respiratory and baroreceptor inputs; whereas, neuronal discharge of the dorsal group near the DVMN is not modulated by either of these inputs. The DVMN group possesses C-fiber axons (conduction velocity, < 2 m s⁻¹) while the NA group has B-fiber axons (conduction velocity, 10 to 30 m s⁻¹). Jones showed that both populations have similar functions (related to cardiac chronotropy, dromotropy and inotropy), although the magnitude and time course of the effects differed substantially and that both populations projected to clusters of ganglion cells on the atrial epicardium.

The study of Jones demonstrates that more than one type of nerve activity exists for communication along an autonomic pathway. Further, that the specific type of activity may, by itself, identify an associated mechanism or group of mechanisms. For example, sensing of high velocity nerve activity may indicate that respiratory and/or baroreceptor mechanisms. An exemplary classification method optionally relies on sensing nerve activity and determining the type of nerve activity (e.g., velocity, frequency or other characteristics).

Referring again to Fig. 4, the device 100 includes connections to leads 110, 110' and 110". The lead 110 includes one or more electrodes 144 for stimulation of a sympathetic pathway 420 while the lead 110' includes one or more electrodes 144' for sensing activity associated with a parasympathetic pathway. The lead 110", which is optional, may acquire information or deliver energy to the cardiopulmonary system (e.g., acquiring cardiac electrograms, intrathoracic impedance, ventricular stimulation, etc.).

In the example of Fig. 4, the device 100 includes logic 238 (see, e.g., the autonomic module 238 of Fig. 2) to cause the device to perform the method 480. The method 480 commences in a start block 482, which may be triggered by a timer, an event, etc. In an acquisition block 484, the method acquires autonomic tone information. For example, the lead 110" may acquire information associated with cardiopulmonary behavior indicative of autonomic tone. Also consider that the leads 110, 110' may be used to sense sympathetic and parasympathetic activity, respectively, to thereby allow for an assessment of autonomic tone. A decision block 486 follows whereby the tone information is used to decide if the tone is OK, for example, bound by some desired limit(s). If the decision block 486 decides that the tone is OK, then the method 480 continues at the start block 482 or takes other appropriate action. However, if the decision block 486 decides that the tone is not OK, then the method 480 continues in an activation block 488 that calls for stimulation of one or more sympathetic afferent pathways. For example, the device 100 may delivery energy to the electrode 144 via the lead 110 to thereby stimulate afferents of the sympathetic pathway 420.

As already mentioned, stimulation of a sympathetic afferent nerve may cause an increase in parasympathetic activity. The activation block 488 may call for stimulation according to one or more stimulation parameters, which may include stimulation sites. Stimulation may occur for only a short period of time to thereby ensure that a global elevation in sympathetic activity does not persist but rather that a parasympathetic response is triggered that persists for some beneficial period of time.

The logic 238 may call for other types of action as alternatives or in addition to the action associated with the method 480. For example, the logic 238 may call for delivery of energy to a sympathetic nerve to block nerve activity (e.g., afferent and/or efferent activity). In a particular example, following activation of sympathetic afferent activity, sympathetic afferent activity is block, which may promote baroreflex function. As baroreflex function can be impaired in patients with heart failure and excessive sympathetic tone, blockade of sympathetic afferent activity may restore this parasympathetic mechanism.

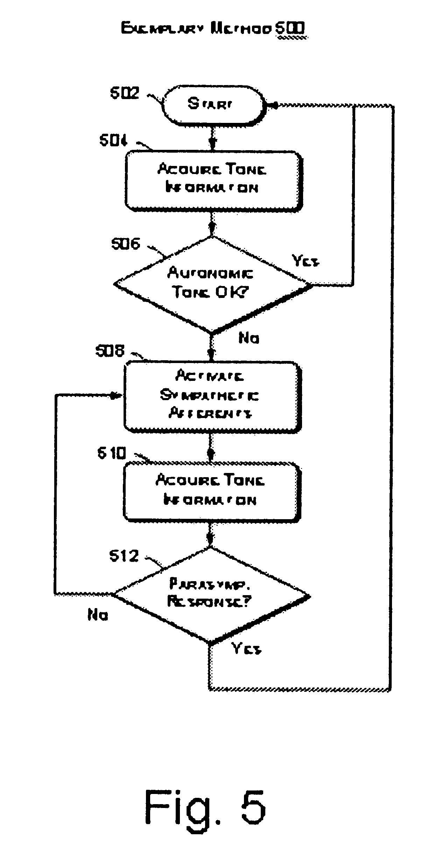

Fig. 5 shows an exemplary method 500 for activating sympathetic afferents and deciding whether a parasympathetic response occurred. The method 500 commences in a start block 502, which may be triggered by a timer, an event, etc. In an acquisition block 504, the method 500 acquires autonomic tone information. A decision block 506 follows whereby the tone information is used to decide if the tone is OK, for example, bound by some desired limit(s). If the decision block 506 decides that the tone is OK, then the method 500 continues at the start block 502 or takes other appropriate action. However, if the decision block 506 decides that the tone is not OK, then the method 500 continues in an activation block 508 that calls for stimulation of one or more sympathetic afferent pathways.

After or during stimulation per block 508, an acquisition block 510 acquires tone information. A decision block 512 follows that uses the tone information to decide whether a parasympathetic response occurred. If the decision block 512 decides that a response did not occur, then the method 500 continues at the activation block 508. However, if the decision block 512 decides that a response did occur or is occurring, then the method 500 continues at the start block 502 or takes other appropriate action.

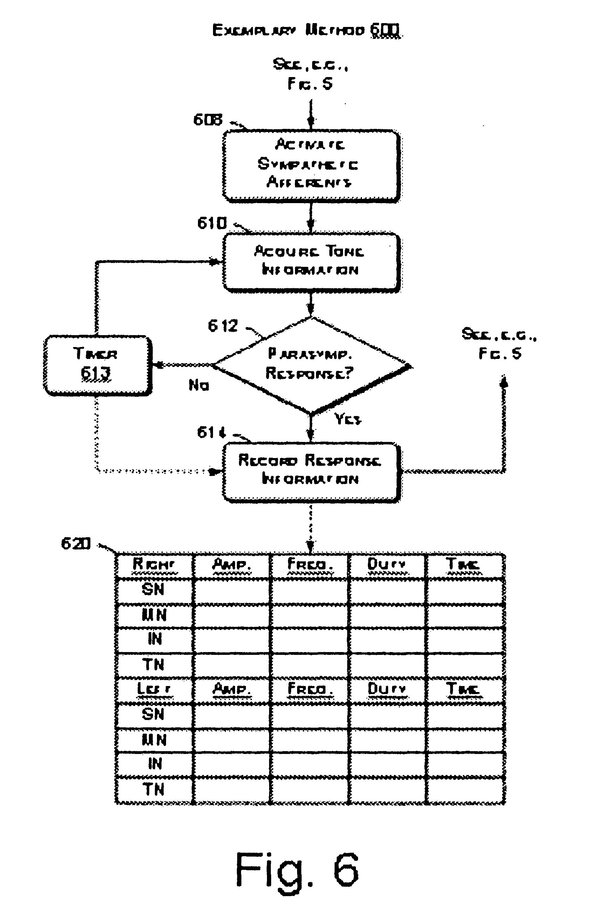

Fig. 6 shows an exemplary method 600 that may be used in conjunction with one or more other methods described herein (e.g., the method 400, the method 500, etc.). The method 600 commences in an activation block 608 that calls for stimulation of one or more sympathetic afferent pathways for a period of time. The method 600 includes an acquisition block 610 that acquires tone information. A decision block 612 uses the tone information to decide whether a parasympathetic response occurred. If the decision block 612 decides that a response did not occur, then the method 600 continues at a timer block 613 that records a time associated with the acquired tone information per block 610 and that optionally implements a delay prior to the method 600 continuing at the acquisition block 610. However, if the decision block 612 decides that a response did occur or is occurring, then the method 600 continues at a recordation block 614 that causes the method 600 to record relevant information pertaining to the activation per block 608 and the parasympathetic response per decision block 612. Further, information from the timer block 613 may also be recorded. After recordation, the method 600 may continue, for example, at a start block (see, e.g., the start block 502).

The recordation block 614 optionally records information as shown in the table 620. The exemplary device 100 or other suitable device optionally stores a table in associated memory (e.g., the memory 260). In the example of Fig. 6, table 620 includes entries for right cardiac nerves and for left cardiac nerves in conjunction with stimulation amplitude, stimulation frequency, stimulation duty cycle, time to parasympathetic response and/or other entries. The stimulation energy or timing of the stimulation energy may be set to reduce or eliminate risk of stimulation to other tissue such as the myocardium, the phrenic nerve, etc.

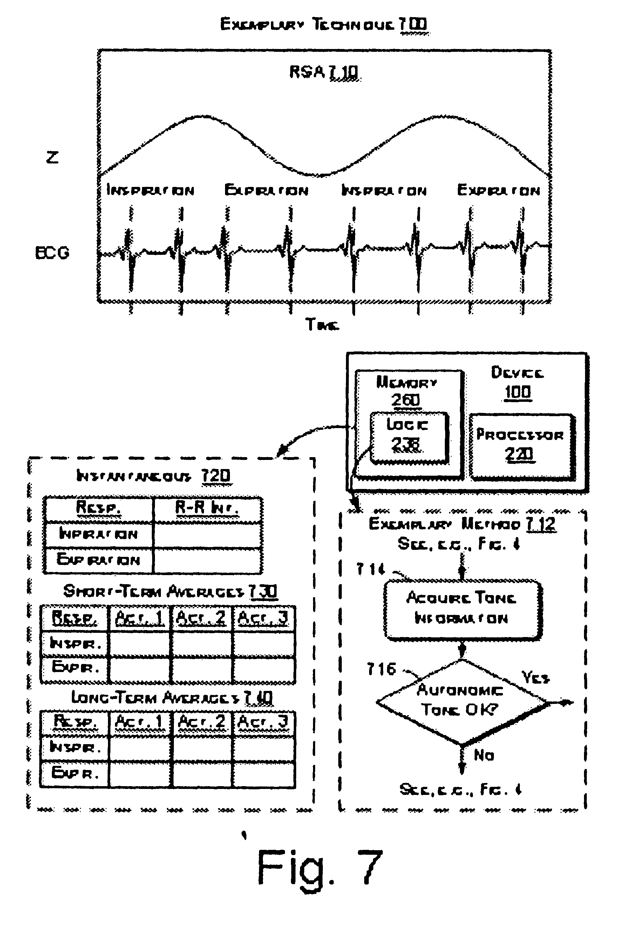

Various methods include acquiring information indicative of autonomic tone. Fig. 7 shows an exemplary technique 700 that acquires respiratory and heart rate information for purposes of assessing autonomic tone. In particular, respiratory sinus arrhythmia (RSA) is known to be caused by inhibition of parasympathetic activity during the inspiratory phase of respiration.
A plot 710 shows an impedance signal that varies with respiratory phase and a cardiac electrogram (e.g., IEGM or other electrogram). The plot 710 shows an increase in heart rate (e.g., shortening of R-R interval) during inspiration and a lengthening during expiration. An exemplary device 100 may acquire such information, store the information in memory 260 where logic 238 may rely on the stored information to assess autonomic tone.

In the example of Fig. 7, the information is organized in tabular form as instantaneous 720, short-term averages 730 and long-term averages 740. While the instantaneous information 720 is likely to be associated with a particular activity state of a patient (e.g., sleep, rest, walking, etc.), the short-term averages 730 and the long-term averages 740 may be organized with respect to a patient's activity state. An exemplary method 710 includes blocks 714 and 716 and the information in tables 720, 730 and 740 may represent acquired information for the acquisition block 714 and a comparison using such information may be performed in the decision block 716. Various other methods described herein may provide for actions prior to and following block 714 and 716.

Fig. 8 shows another exemplary technique 800 for acquiring information to assess autonomic tone. Fig. 8 includes an approximate anatomical diagram that shows various parasympathetic pathways including the right branch 880 (X_{right}) and the left branch 890 (X_{left}) of the tenth cranial nerve (X) also known as the vagus nerve or vagal nerve. The vagal nerve is part of the autonomic system and regarded primarily as a parasympathetic nerve. The aforementioned article by Kawashima categorized vagal cardiac branches with direct connections or connections via the cardiac plexus, excluding branches of the lung or surrounding vessels and organs, as follows: superior cardiac branch (SB), which arose from the vagus nerve at about the level of the upper (proximal) portion of the recurrent laryngeal nerve branch (RL); inferior cardiac branch (IB), which arose from the recurrent laryngeal nerve branch (RL); and thoracic cardiac branch (TB), which arose from the vagus nerve at about the level of the lower (distal) portion of the recurrent laryngeal nerve branch (RL).

Fig. 8 shows approximate locations of some branches of the right vagus nerve 880 (SB 881; RL 882; IB 883; TB 884; TN 885) and the left vagus nerve 890 (SB 891; RL 892; IB 893; TB 894), with respect to the superior vena cava 160, the brachiocephalic trunk 166, the trachea 164 and the aortic arch 168. The dashed lines indicate that the right vagal nerve 880 and its various branches are not in the fore of the diagram but rather lie generally aft (dorsal) of the SVC 160. For the left vagus nerve 890, the path courses fore (ventral) of the aortic arch 168 where a branch or branches pass underneath the arch and continue to various regions. Further the dashed lines do not indicate any particular length but rather a general course of such branches as they extend to, or around, the heart and other structures.

In Fig. 8, an exemplary device 100 includes a processor 220, memory 260 and logic 238. The device 100 is operably connected to one or more electrodes 144', for example, via a lead 110'. In this arrangement, parasympathetic nerve activity may be sensed along the left vagus 890 (X_{left}) as an indicator of autonomic tone (or simply parasympathetic activity). An exemplary method 810 includes blocks 814 and 816 where the device 100 acquires information for the acquisition block 814 and the logic 238 provides for decision making in the decision block 816. Various other methods described herein may provide for actions prior to and following block 814 and 816.

Referring again to Fig. 4, the approximate anatomical diagrams of Figs. 3 and 8 may be used for any of a variety of purposes including selection of stimulating and/or sensing sites for autonomic nerves. With respect to sensing and/or stimulating autonomic nerves, various types of electrodes exist. For example, cuff electrodes are commonly used for sensing and/or stimulating. In particular, an electrode known as a spiral cuff electrode is suitable for placement on an autonomic nerve. Electrode arrays may also be used. For example, an electrode array may be configured as a cuff or a plurality of cuffs. Individual electrodes in an array or groups of electrodes in an array may be selected as appropriate through use of techniques such as the switching circuitry 226 of Fig. 2.

According to various exemplary technologies described herein, a pulse, a series of pulses, or a pulse train, can be delivered via an electrode-bearing lead portion, for example, operably connected to an implantable device to thereby activate an autonomic nerve, other nerve or tissue. The exemplary electrode-bearing lead portion may be used to selectively activate a nerve or optimally activate a nerve through its configuration and optionally through selection of and polarity of one or more electrodes.

A pulse or pulse train optionally includes pulse parameters or pulse train parameters, such as, but not limited to, duty cycle, frequency, pulse duration (or pulse width), number of pulses or amplitude. These parameters may have broad ranges and vary over time within any given pulse train. In general, a power level for individual pulses or pulse trains is determined based on these parameters or other parameters.

Exemplary ranges for pulse frequency for nerve or tissue stimulation include frequencies ranging from approximately 0.1 to approximately 100 Hz, and, in particular, frequencies ranging from approximately 1 Hz to approximately 20 Hz. Of course, higher frequencies higher than 100 Hz may also be suitable. Exemplary ranges for pulse duration, or pulse width for an individual pulse (generally within a pulse train), include pulse widths ranging from approximately 0.01 milliseconds to approximately 5 milliseconds and, in particular, pulse widths ranging from approximately 0.1 milliseconds to approximately 2 milliseconds. Exemplary pulse amplitudes are typically given in terms of current or voltage; however, a pulse or a pulse train may also be specified by power, charge and/or energy. For example, in terms of current, exemplary ranges for pulse amplitude include amplitudes ranging from approximately 0.02 mA to approximately 20 mA, in particular, ranging from 0.1 mA to approximately 5 mA. Exemplary ranges for pulse amplitude in terms of voltage include voltages ranging from approximately 2 V to approximately 50 V, in particular, ranging from approximately 1 V to approximately 20 V.

As described herein, various exemplary methods, devices, systems, etc., include nerve stimulation, for example, to promote parasympathetic activity or to balance autonomic tone. Depending on electrode location, stimulation parameters, etc., some risk may exist for undesirable myocardial stimulation. Undesirable myocardial stimulation generally includes stimulation that may interfere with proper operation of the heart. For example, delivery of stimulation during a vulnerable period may cause arrhythmia. To avoid undesirable myocardial stimulation and/or to reduce risk associated with any inadvertent myocardial stimulation associated with stimulation of a nerve, various exemplary methods, devices and/or systems include or can implement timing and/or pacing schemes. For example, an exemplary method includes synchronizing delivery of a nerve stimulation pulse train with the action potential refractory period of a myocardium depolarization, which may be due to a paced and/or an intrinsic event.

According to various exemplary methods, devices and/or systems described herein, and equivalents thereof, stimulation of autonomic nerves, other nerves and/or tissue allows for influence of cardiac activity. For example, various exemplary methods and corresponding stimulation devices rely on placement of one or more electrodes in a vessel, e.g., an epicardial vein or an epicardial venous structure. Suitable epicardial veins or venous structures include the coronary sinus and veins that drain into the coronary sinus, either directly or indirectly. For example, the great cardiac vein passes along the interventricular sulcus, with the anterior interventricular coronary artery, and empties anteriorly into the coronary sinus; and the middle cardiac vein travels with the posterior (right) interventricular coronary artery and empties into the coronary sinus posteriorly. Other suitable veins include those that drain into the right atrium or right auricle. For example, the anterior cardiac vein passes through the wall of the right atrium and empties into the right atrium.

Other exemplary methods, devices, systems, etc., rely on placement of one or more electrodes in a non-epicardial vein. Such exemplary methods, devices, systems, etc., are optionally suitable for stimulation of autonomic nerves at locations, for example, generally along an autonomic pathway between the heart and brain. Further, other exemplary methods, devices and/or systems rely on placing one or more electrodes through the wall of a vein and proximate to an autonomic nerve, other nerve or tissue. Yet other exemplary methods, devices, systems, etc., rely on placing one or more electrodes proximate to a nerve without first passing the electrode through a vein or vein wall.

Another type of placement for an electrode and/or lead involves epicardial via the intrapericardial space from outside of the pericardial sac. For example, a subxyphoid incision and insertion of a needle, stick or other placement device may be made to access the pericardial sac (e.g., a process used for pericardiocentesis) and to position an electrode and/or lead. Such an electrode or lead may then be connected to an implantable device (e.g., the device 100). In some instances, a small satellite device may be implanted in the intrapericardial space where the satellite device communicates (uni- or bidirectional) with another device (e.g., the implantable device 100).

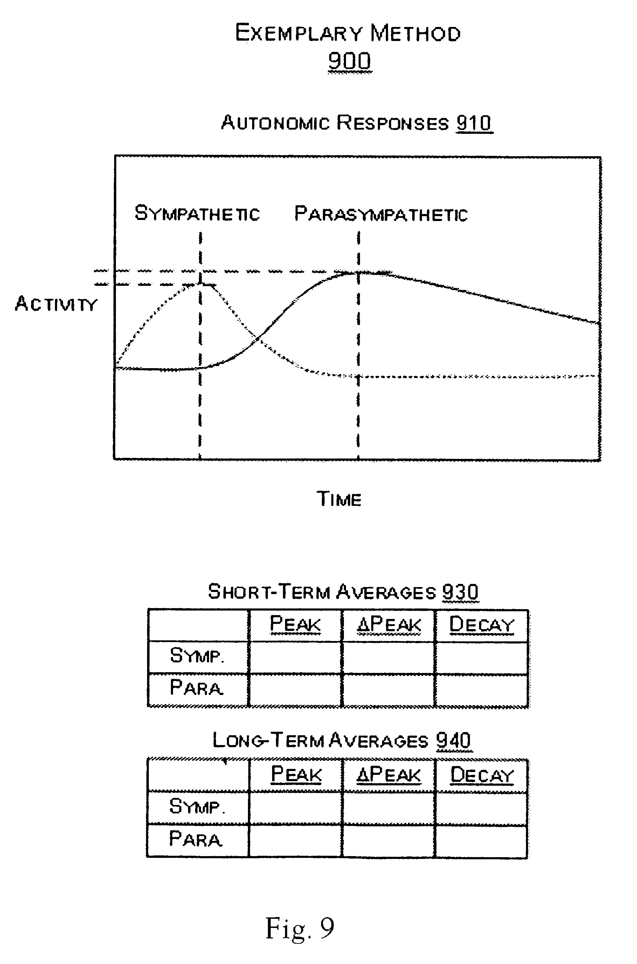

Fig. 9 shows an exemplary method 900 for analyzing autonomic responses. As already mentioned, short-term activation of a sympathetic afferent pathway can cause an increase in parasympathetic activity. A plot 910 shows a rise in sympathetic activity in response to activation of a sympathetic afferent pathway and a delayed rise in parasympathetic activity. Given appropriate sensing equipment, autonomic information may be acquired and stored. For example, Fig. 9 includes a short-term average table 930 and a long-term average table 940. The tables 930, 940 include entries for peak time, peak delta (e.g., a rise from a baseline activity value) and a decay for a time constant or other indicator as to time decay of activity following a rise in activity. Such tables may be used to assess patient condition (e.g., analysis for trends, etc.). For example, given a subject with heart failure, the rise in parasympathetic may decrease as heart failure worsens (e.g., NYHA class III to class IV) or the decay in parasympathetic activity may be faster or the rise delayed as heart failure worsens. Where a device provides for cardiac pacing or other cardiac therapy, such information may be used to adjust cardiac therapy.

An exemplary method includes delivering electrical stimulation to a sympathetic afferent nerve, measuring parasympathetic nerve activity responsive to the electrical stimulation of the sympathetic nerve and assessing patient condition based at least in part on the measured parasympathetic nerve activity. Such a method is optionally implemented as instructions on a computer-readable medium suitable for execution by a processor (see, e.g., the microprocessor 220 of Fig. 2). Such a method optionally includes adjusting one or more parameters of a cardiac therapy based at least in part on an assessed patient condition. Other types of therapies may also benefit from such a method (e.g., respiratory therapies, vagal stimulation therapies, etc.).

Various exemplary techniques discussed herein recognize that a link exists between sympathetic and parasympathetic nerve activity. Various exemplary techniques include stimulation of afferent sympathetic nerve(s) to help restore or address autonomic balance and improve patient condition. While various exemplary techniques can be automated via control logic and processor, a patient or clinician may be able to manually trigger stimulation of an afferent sympathetic nerve (e.g., through use of a magnet and magnet detection circuitry, telemetry, etc.). As already mentioned, such nerve stimulation may occur transvenously, endocardially, by direct nerve stimulation, etc. For example, one or more nerve cuff electrodes may be placed at the cerviocothoracic (stellate) ganglion, the subclavian ansa, etc. An exemplary technique may use specialized electrodes and lead systems placed at a plexus or inside a vein (e.g., SVC, CS, etc.). A technique may optionally use a standard RV defibrillation and/or pacing lead for stimulation of the afferent sympathetic neurons innervating the ventricles (see, e.g., the lead 108 of Fig. 1).

Various exemplary techniques include control logic that may respond to certain conditions. Such conditions may be determined using acquired information (e.g., acquired via sensing, telemetry, etc.). An exemplary device may be capable of measuring efferent sympathetic and/or parasympathetic nerve activity directly through sensing nerve firing. As already mentioned, indirect assessment of autonomic tone may rely on RSA or heart rate variability (HRV), etc. An exemplary device may detect (directly or indirectly) abnormally high sympathetic activity or low parasympathetic efferent activity and, in response, trigger an algorithm that calls for stimulation of sympathetic afferent neurons.

Various exemplary techniques aim to promoting sympathetic afferent activities to increase the global vagal tone to alleviate (e.g., improve) conditions such as heart failure, ischemia, and arrhythmia. Various exemplary techniques can selectively promote sympathetic afferent activities. Various exemplary techniques may include delivering nerve stimulation sub-threshold (to avoid muscle contraction) and/or sub-perception stimulation (to avoid sensation, pain, etc.).

## Claims

1. A system comprising: means (222, 224) for delivering electrical stimulation to a sympathetic afferent nerve; means for acquiring information (244, 246) indicative of autonomic tone; and means for determining (238), based at least in part on the information, if the delivering caused an increase in parasympathetic nerve activity.

2. A system as claimed in Claim 1, **characterised in that** the means for delivering (222, 224) delivers electrical stimulation (222, 224) to a left side sympathetic afferent nerve (390), or to a right side sympathetic afferent nerve (380).

3. A system as claimed in Claim 1, **characterised in that** the means for delivering (222, 224) delivers electrical stimulation to at least one nerve selected from superior cardiac nerves (380, 391), middle caridac nerves (382, 392), inferior cardiac nerves (383, 393), and thoracic cardiac nerves (384, 394).

4. A system as claimed in Claim 1, **characterised in that** the means for delivering (222, 224) delivers electrical stimulation to a nerve other than the left thoracic cardiac nerve (394).

5. A system as claimed in any preceding claim, **characterised in that** the means for acquiring (224, 246, 270) acquires heart rate and/or a measure of respiration and/or parasympathetic nerve activity.

6. A system as claimed in any preceding claim, **characterised in that** it comprises an implantable cardiac electrical stimulation device (100).

7. A system as claimed in Claim 6, **characterised in that** the means for delivering comprises a pulse generator (222, 224) and the means for acquiring comprises sensing circuitry (244, 246).

8. A system as claimed in Claim 6 or Claim 7, **characterised in that** the device comprises: a processor (222), a memory (260) and control logic, the control logic being implemented through use of the processor (222) to call for the delivery of electrical stimulation to the sympathetic afferent nerve (380, 390), to call for the acquiring of information indicative of autonomic tone and to determine, based at least in part on the information, if the delivering caused an increase in parasympathetic nerve activity.

9. A system as claimed in any of Claims 6 to 8, **characterised by** connectors to electrically connect an electrode-bearing lead (104, 106, 108, 110) to the device.

10. A system as claimed in Claim 9, **characterised in that** the lead comprises a spiral electrode positionable on an autonomic nerve.

11. A system as claimed in any of Claims 6 to 10, **characterised in that that** control logic is organised to adjust a cardiac pacing therapy based at least in part on whether the delivering caused an increase in parasympathetic nerve activity.

12. A system as claimed in any of Claims 6 to 11, **characterised in that** the control logic is arranged to set a time period and if the determining means does not determine that the delivering caused an increase in parasympathetic nerve activity within the time period, then the control logic is arranged to adjust one or more stimulation parameters.

13. A system as claimed in Claim 12, **characterised in that** the stimulation parameter is selected from amplitude, frequency, duty cycle, stimulation site, and polarity.
